# EUROPEAN PATENT SPECIFICATION

(11) **EP 0 206 533 B1**
(45) Date of publication and mention of the grant of the patent: **13.04.1994**
(21) Application number: 86303955.8
(22) Date of filing: 23.05.1986
(51) Int. Cl.: C07K 15/28, C12P 21/08

(54) **Platelet inhibiting monoclonal antibody fragment**
Plättcheninhibierender Teil eines monoklonalen Antikörpers
Fragment d'anticorps monoclonal inhibant les plaquettes

(30) Priority: 14.06.1985 US 745415; 14.04.1986 US 851711
(43) Date of publication of application: 30.12.1986
(73) Proprietor: THE RESEARCH FOUNDATION OF STATE UNIVERSITY OF NEW YORK, Albany, New York 12201-0009 (US)
(72) Inventor: Coller, Barry S., Dix Hills New York 11746 (US)
(74) Representative: Ford, Michael Frederick

(56) References cited:
- US-A- 4 489 710
- CHEMICAL ABSTRACTS, vol. 104, no. 3, 20th January 1986, page 345, abstract no.18049y, Columbus, Ohio, US; V.T. LOMBARDO: "Independent modulation of von Willebrand factor and fibrinogen binding to the platelet membrane glycoprotein IIb/IIIa complex as demonstrated by monoclonal antibody"
- PROC. NATL. ACAD. SCI. USA, vol. 82, May 1985, pages 3465-3468, Medical Sciences, US; Z.H. OSTER et al.: "Thrombus radioimmunoscintigraphy: An approach using monoclonal antiplatelet antibody"
- CHEMICAL ABSTRACTS, vol. 104, no. 9, 3rd March 1986, page 546, abstract no.67189c, Columbus, Ohio, US; B.S. COLLER et al.: "Inhibition of dog platelet function by in vivo infusion of F(ab)2 fragments of a monoclonal antibody to the platelet glycoprotein IIb/IIIa receptor"
- CHEMICAL ABSTRACTS, vol. 105, no. 11, 15th September 1986, page 298, abstract no.93713j, Columbus, Ohio, US; P. SOM et al.: "Technitium-99m labeled antiplatelet monoclonal antibody fragments (MAP-Fab) for radioimmunoimaging of experimental thrombi"
- CHEMICAL ABSTRACTS, vol. 103, no. 13, 30th September 1985, Page 416, abstract no.102626w, Columbus, Ohio, US; B.S. COLLER et al.: "A new murine monoclonal antibody reports an activation-dependent change in the conformation and or microenvironment of the platelet glycoprotein IIb/IIIa complex"
- JOURNAL OF NUCLEAR MEDECINE, vol. 26, no. 4, April 1985, page 439; M.L. THAKUR et al.:"Consideration for the preparation of radiolabeled monoclonal antibodies with high specific activity"
- CHEMICAL ABSTRACTS, vol. 98, no. 13, 28th March 1983, page 461, abstract no.105520w, Columbus, Ohio, US; E. LAMOYI et al.: "Preparation of F(ab)2 fragments from mouse IgG of various subclasses"
- Coller et al. (1983) J. Clin. Invest. 72:325-338
- Yasuda et al. (1988) J. Clin. Invest. 81:1284-1291

## Description

Blood platelets, also known as thrombocytes, function in the human body to provide for the coagulation of blood when the blood supply is exposed to potential loss, i.e., trauma, abrasions, and other hemorrhaging injuries. Platelets rapidly interact with damaged blood vessels and with each other to prevent the escape of blood cells and also facilitate the formation of a blood clot in which they become enmeshed so as to plug up any "leaks" in the vascular system. However, in certain disease states, such as thrombosis, it is desirable and even necessary, to inhibit blood platelet function. This is because the thrombus can either occlude the blood vessel and cause damage to the tissue being supplied with blood from that vessel (for example, myocardial infarction, stroke after vascular surgery, etc) or because the platelets and other thrombotic material can break off from the thrombus and lodge down-stream in blood vessels supplying other parts of the body (for example, venous thrombosis with pulmonary embolism, transient ischemic attacks, amaurosis fugax, strokes from the artificial hearts, etc).

Drugs currently utilized to counteract the platelet function suffer from a lack of potency and/or specificity. Thus, there is a continuing need for new and improved drugs that will inhibit platelet function in diseases characterized by thrombosis.

The present invention concerns a monoclonal antibody fragment which inhibits platelet function. The fragment was produced by the proteolytic digestion of the 7E3 monoclonal antibody, suitably with papain or pepsin. The 7E3 monoclonal antibody was produced in the following manner. Human blood platelets were injected into mice. The mouse spleen was removed and fused with a mouse myeloma by a modification of the technique of Levy et al., (Curr. Top. Microbiol. Immunol., 81, 164 (1978)). The fused cells were incubated and then further incubated in HAT medium (in which non-fused cells will not grow). The cells were then diluted out and screened in a screening assay for antifibrinogen receptor activity. There was selected one particular clone, designated 7E3 of the class IgG₁ which reacts with normal human blood platelets and with dog blood platelets, fails to react with thrombasthenic platelets or human platelets whose GPIIb/IIIa complex was disassociated with EDTA, reacts slowly with unactivated human platelets and more rapidly with ADP activated human platelets and completely blocks the interaction of fibrinogen with platelets induced by ADP.

Coller B.S. et al in J. Clin. Invest. (1983) Vol.72 p.325-338 describes a monoclonal antibody 10E5 of the IgG2a subclass. This 10E5 antibody was produced by intraperitoneal injections of a Balb/c mouse with human blood platelets. Spleen cells from the mouse were fused with the nonsecretory Balb/c mouse myeloma cell line X63-Ag 8.653. The 10E5 antibody and F(ab')₂ fragments thereof are described as blocking the binding of fibrinogen to the platelet surface. No therapeutic use is proposed for the 10E5 antibody.

Oster Z.H. et al in PNAS (1985) Vol.82 p.3465-3468 describes a whole monoclonal antibody 7E3 which inhibits interaction between fibrinogen-coated beads and both human and dog platelets. The 7E3 hybridoma was made according to the technique described in Coller B.S. et al 1983 supra. No disclosures were made concerning fragments of the 7E3 antibody and of any therapeutic use of the antibody.

The Balb/c mouse myeloma cell line X63-Ag 8.653 is described in Kearney J.F. et al J. Immunol. 1979 Vol.123 p.1548-1550.

Kornecki et al, (1984) Thrombosis Research 34: p.35-49 discloses a mouse monoclonal antibody MA123 which inhibits the binding of fibrinogen to ADP-stimulated human platelets. However MA123 does not react with dog blood platelets.

The Figures I, II and III respectively show as
a) the plots of the change in platelet-rich plasma optical density one minute after ADP addition to blood previously treated with the F(ab')₂ fragments at given previous intervals.
b) a plot of molecules of ¹²⁵I-7E3 bound per platelet (X 10⁻³) at certain time intervals after administration of the fragment.

Figure IV (a-d) is a continuous set of plots for one test dog of aortic blood pressure and circumflex blood flow against time after administration of the F(ab')₂ fragments.

Figure V is an optical density/time plot of ADP response pre and post F(ab')₂ infusion.

### a) Preparation

7E3 Monoclonal antibody was prepared according to the procedures set forth below:
Citrated platelet-rich plasma were prepared in accordance with the method of Coller et al., (Blood, 47, 841 (1976)), suspended in a suitable buffer and mixed with Freund's complete adjuvent. Injections of between about 1 x 10⁸ to 5 x 10⁸ washed platelets were injected six times at weekly intervals into a BALB/c mouse intraperitoneally and a seventh similar injection without adjuvant at a similar time interval was given intravenously. Three days later the mouse was killed, the spleen removed, the cells separated and fused with a BALB/c mouse myeloma line in accordance with the method of Levy et al., (supra). In this method, the spleen cells and the myeloma cells in a ratio of 3.9:1 were pelletted together, the pellet suspended in polyethylene glycol (35%) in RPMI 1640 medium whereupon the cells were immediately centrifuged at low velocity. The solution was then diluted to about 25% of its previous concentration with RPMI 1640, the cells resuspended, recentrifuged and the supernatant removed. The supernatant was then incubated in a 5% CO₂ 95% air atmosphere in RPMI 1640 medium supplemented with fetal calf serum and thereafter selection made in the usual manner by adding HAT medium and aliquoting into microtiter wells. After two weeks, the supernatant of the wells that showed growth were screened for antifibrinogen receptor activity. The clones obtained by this method were selected for various qualities; one in particular, 7E3 was selected for certain qualities.

The antibody was isolated from the supernatant in the wells or flasks. Alternatively, the hybridomas were injected intraperitoneally into Pristane^{R} pretreated BALB/c rats and the antibodies isolated from the ascitic fluid. The antibody was purified by precipitation with 50% saturated ammonium sulfate, resuspended in between 5 and 10% of the original volume in sodium phosphate buffer and dialyzed against the same buffer. Chromatography on protein A-Sepharose CL-4B equilibrated with phosphate buffer was carried out, elution was with phosphate buffer followed by decreasing pH 0.1M citrate buffers. 7E3 antibody was eluted after the pH decreased to about 6.0. Protein elution was as monitored by ultra-violet spectroscopy at 280 nm.

Ouchterlony immuno diffusion analysis against anti-IgG1, IgG2a, IgG2b, IgG3, IgM and IgA sera indicated the exclusive presence of IgG1.

Purified antibody was then dialyzed overnight at at reduced temperatures, suitably between about 0 and 10°C preferrably at 4°C against a slightly acidic saline buffer of pH 3.5-6.5, suitably about pH 4.0, after which the freshly prepared pepsin was added in an amount equal to approximately 2% of the antibody's weight. The resulting solution was then incubated at about 37°C for 12 to 24 hours. Digestion was stopped by dialyzing the solution against PBS, pH 7.4. Analysis by polyacrylamide gel electrophoresis indicated that the digestion was essentially complete.

Under somewhat different conditions, an Fab fragment can be prepared by digestion with papain, another proteolytic agent, namely, .1M acetate, 2mM EDTA, 1mM cysteine and including 1% w/w of papain for 6-8 hours at 37° at a pH of 4.5-6, suitably 5.5.

The resulting F(ab')₂ fragment of the 7E3 monoclonal antibody can then be further purified by chromatography on a material such as protein A Sepharose CL-4B or DE-52 in order to be certain that any remaining traces of the whole 7E3 monoclonal antibody are removed.

### EXAMPLE I

### Antibody Production

A BALB/c mouse (Jackson Laboratories, Bar Harbor, Me.) was injected intraperitoneally with six weekly 0.2 ml injections of 3 x 10⁸ washed platelets (citrated PRP washed twice in 0.15 M NaCl, 10 mM Tris/Cl, 10 mM EDTA, pH 7.4 [TS-E]), resuspended in 1/10 to 1/20 of their original volume in TS-E, and mixed 1:1 with complete Freund's adjuvant. The seventh weekly injection was given intravenously into the tail vein and consisted of 0.3 ml. containing 5 x 10⁸ washed platelets resuspended in T-S without EDTA. Each of the seven platelet suspensions was obtained from a different donor. Three days after the last injection, the mouse was killed by cervical dislocation and the spleen removed. A suspension of spleen cells in RPMI 1640 was prepared by teasing the spleen apart. After erythrocytes were lysed with ammonium chloride, the spleen cells were fused with a nonsecretory BALB/c mouse myeloma cel line (X63-Ag 8.653) that had been kept frozen in 10% DMSO, 90% fetal calf serum until one week before fusion, when it was thawed and maintained in the culture medium routinely used (RPMI 1640 supplemental with 10% fetal calf serum and 1,000 U of penicillin and 100 ug of streptomycin/ml). Fusion was carried out according to a modification of the method of Levy et al. (supra). Briefly, 2.7 x 10⁸ spleen cells and 7 x 10⁷ myeloma cells were pelleted together, the pellet was gently suspended in 2 ml of 35% polyethylene glycol in RPMI 1640 medium and the cells immediately centrifuged at 500 g at 22°C for 6 minutes. The solution was then diluted with RPMI 1640 to 9% polyethylene glycol, the cells resuspended and immediately centrifuged at 230 g. for 6 minutes at 22°C. The supernatant fluid was then aspirated and the fused cells suspended in RPMI 1640 medium and supplemented with 20% fetal calf serum and 10% 109 medium (National Collection of Type Cultures). The cells were placed in a flask and incubated overnight at 37°C in a 5% CO₂, 95% air atmosphere. The following day, the medium was made selective for successfully hybridized cells by adding hypoxanthine (10⁻⁴ M), aminopterin (4 x 10⁻⁷ M), and thymidine (1.6 x 10⁻⁵ M), after which the cells were aliquoted into 960 microtiter wells (Costar, Data Packaging, Cambridge, Ma.). Two weeks later, 574 wells showed growth and the supernatant fluids from 59 wells were positive in a screening assay for antifibrinogen receptor activity (see below). After an additional two weeks in culture, the positive clones were transferred to 24-well microtiter dishes (Costar) and fed with the same medium as above, but without the aminopterin. The clones were expanded and the cells that continued to produce antifibrinogen receptor antibody were suspended in 90% fetal calf serum-10% DMSO and frozen in liquid nitrogen.

The clones were subcloned by both limiting dilution technique and growth in soft agar to insure monoclonality.

Ascitic fluid rich in 7E3 antibody was prepared by intraperitoneal injection of Pristane-pretreated BALB/c mice with 5 x 10⁶ hybrid cells that had been washed twice in 0.15 M NaCl, 10 mM sodium phosphate, pH 7.4 (PBS).

### EXAMPLE II

### Screening Assay

35 ul of PRP (platelet rich plasma) (adjusted to 3 x 10¹¹ platelets/liter) and 35 ul. of the supernatant culture medium (or ascitic fluid) to be assayed were incubated together for 2-60 minutes in a well of a round-bottomed microtiter plate (Linbro Chemical Co., Hamden, Ct). 5 ul of the fibrinogen-coated bead suspension was then added and the plate was mixed on a rotator (Tekator V, American Scientific Products, Edison, N.J.) for 5 minutes at 280 rpm. The wells were observed from the bottom with the aid of a magnifying mirror apparatus (Cooke Microtiter System, Dynatech Laboratories, Inc., Alexandria, Va.). Wells containing culture medium that had not been used for growing cells showed marked agglutination of the beads (rated as 4+), whereas the supernatant culture medium or mouse ascitic fluid from positive clones inhibited the agglutination, resulting in lower readings (0-3+).

### EXAMPLE III

### Antibody Purification

Culture supernatants were precipitated at 4°C with 50% saturated ammonium sulfate and resuspended to between 1/20 and 1/10 of their original volume in 0.1 M sodium phosphate buffer, pH 8.0. After dialysis against the same buffer, the samples were applied to a 0.8 x 15.9 cm column of protein A Sepharose CL-4B that had been equilibrated with the phosphate buffer (after having been washed with both the phosphate buffer and a 0.1 M citrate buffer, pH 3.0). The column was eluted with the phosphate buffer until the optical density of the eluate returned to base line, after which stepwise elution was accomplished with 0.1 M citrate buffers of pH 6.0, 4.5, 3.5 and 3.0, as described by Ey, et al., (Immunochemistry, 15, 429 (1978). 7E3 immunoglobins were eluted at pH 6.0. Protein elution was monitored by optical density at 280 nm and appropriate fractions were pooled and dialyzed against T-S containing 0.05% sodium azide. Antibody concentration was estimated by absorption at 280 nm, assuming A^{1%} = 15.

### EXAMPLE IV

### Preparation of Fragment

7E3 Monoclonal antibody was prepared according to the procedures disclosed in the foregoing examples.

Purified antibody was then dialyzed overnight at approximately 4°C against 0.2M sodium chloride, 0.2M acetate, pH 4.0, after which freshly prepared pepsin (1 mg/ml) was added in an amount equal to approximately 2% of the antibody's weight. The resulting solution was then incubated at about 37°C for 12 to 24 hours. Digestion was stopped by dialyzing the solution against PBS, pH 7.4. Analysis by polyacrylamide gel electrophoresis indicates that the digestion was essentially complete.

The resulting F(ab')₂ fragment of the 7E3 monoclonal antibody can then be further purified by chromatography on a material such as protein A Sepharose CL-4B or DE-52 in order to be certain that any remaining traces of the whole 7E3 monoclonal antibody are removed.

### EXAMPLE V

### Testing

The F(ab')₂ monoclonal antibody fragment was then tested for platelet function inhibition by the following method:
Three dogs were utilized. Dog No. 1 received a total of 0.17 mg/kg. in three injections given one hour apart. A blood sample drawn one hour after the last injection showed the following: 1) had a 61% decrease in the maximum extent of aggregation (Tmax) induced by 4 mADP; 2) having a platelet count of 220,000/ul (control (t = 0)) = 236,000; and 3) bound 45,400 molecules/platelets of ¹²⁵I-7E3 (control = 56,400), which suggests that 11,000 molecules of the F(ab')₂ fragment of the 7E3 monoclonal antibody are bound per platelet. Dog No. 2 received a single 0.57 mg/kg dose. 1.5 Hours later, the Tmax to ADP had decreased by 68% and the number of molecules of ¹²⁵I-7E3 bound/platelet had decreased by 59% (from 62,500 to 25,400); at 4 hours there was an 84% reduction in Tmax and a 64% reduction in molecules bound (22,500). The initial platelet count of 265,000 decreased to 253,000 at 1.5 hours and to 213,000 at 4 hours, but returned to 242,000 at 20 hours. Neither dog bled spontaneously.

A third dog received 0.8 ug/kg of fragment as shown in Figure III. These test results indicate that the F(ab')₂ fragment of the 7E3 monoclonal antibody can dramatically inhibit platelet function in vivo without causing profound thrombocytopenia.

It will be apparent to those skilled in the art that many modifications, both of materials and methods, may be practiced without departing from the purpose and intent of this disclosure.

### EXAMPLE VI

### In Vivo Testing

The cyclical blood flow reduction model has previously been described in detail (Folts, J.D., E.B. Crowell, Jr., and G.G. Rowe, 1976, Platelet Aggregation in Partially Obstructed Vessels and its Elimination with Aspirin, Circulation 54:365-370; Folts, J.D., 1982, Experimental Arterial Platelet Thrombosis, Platelet Inhibitors and their Possible Clinical Relevance, Cardiovasc. Rev. Rep., 3:370-382; Folts, J.D., Gallagher, K.., and Rowe, G. G., 1982, Blood Flow Reductions in Stenosed Canine Cornary Arteries: Vasopasm or Platelet Aggregation , Circulation 65:248-255), the disclosure of which is incorporated herein by reference. Briefly, either the circumflex coronary artery of an anesthetized dog or the carotid artery of an anesthetized monkey is dissected out and an electromagnetic probe is placed on it to measure blood flow. The artery is stenosed approximately 70% by placing a 3-4 mm long encircling plastic cylinder of the appropriate internal diameter around the outside of the artery. Cyclical reductions in blood flow due to platelet thrombus formation then commence whether spontaneously or after additional intimal damage is produced by brief (ca. 1 sec.) clamping of the artery with a hemostat. Within several minutes, blood flow in the vessel decreases to a critical level, after which the flow is abruptly restored to its original level, either by spontaneous embolization of the platelet thrombus, or by embolization induced by gently shaking the cylinder. Once established, the cyclical flow reductions persist if no intervention is made. The frequency and amplitude of the flow reductions can be temporarily enhanced by infusing 0.5 ug/kg/min. of epinephrine intravenously for 10 min.

The experimental protocol consisted of first obtaining samples of blood anticoagulated with either 0.01 volume 40% sodium citrate (for platelet aggregation and ¹²⁵I-7E3 binding), or 0.037 volume 269 mM EDTA (for platelet counts), and then performing the surgery. After cyclical flow reductions were established, another set of blood samples was obtained and the antibody (0.7-0.8 mg/kg) was infused intravenously as a bolus. A final set of samples was obtained 30 min. after infusing the antibody. Platelet counts on the platelet-rich plasma were performed by microscopy after dilution and lysis of erythrocytes (Unopette, Becton-Dickinson), and whole blood platelet counts were performed by an electronic resistive particle counter (S+IV, Coulter Electronics, Hialeah, FL.).

### Results

Cyclical reductions in blood flow were obtained in 4 dogs and 4 monkeys. In all 4 dogs and 4 monkeys, infustion of the F(ab')₂ fragments resulted in complete cessation of the cyclical flow reductions and restoration of the control flow rate in 10 min. or less (Figure IV). In the dogs, restoration of coronary flow correlated with the disappearance of ST segment deviations on the electrocardiograms, indicating the reversal of myocardial ischemia. The cyclical flow reductions could not be restored with epinephrine infusions of 0.5 or 1 ug/kg/min. for 10 minutes, increasing the intimal damage by repeated brief clamping of the vessel with a hemostat, passing current (1-2 mA) through the cylinder, or a combination of these provocations (Figure IV). Postmortem histologic examination of the blood vessels confirmed the presence of extensive intimal damage.

Platelet aggregation in response to ADP (≦25 uM) was virtually abolished by the antibody infusion in all 8 animals, although the shape change response remained intact (Figure V). ¹²⁵I-7E3 antibody binding studies were performed on 1 dog and 1 monkey. In both animals 84% of the GPIIb/IIIa receptors were blocked by the F(ab')₂ fragments (dog = 34,300 molecules of ¹²⁵I-7E3 bound per platelet before infusion and 5,500 after; monkey = 49,700 before and 8,100 after). Platelet counts decreased by less than 15% in the 3 animals tested. None of the animals demonstrated a significant change in heart rate or blood pressure upon antibody infusion. There was no clear evidence of excessive hemorrhage from the operative sites after antibody infusion, although no objective data on blood loss were obtained.

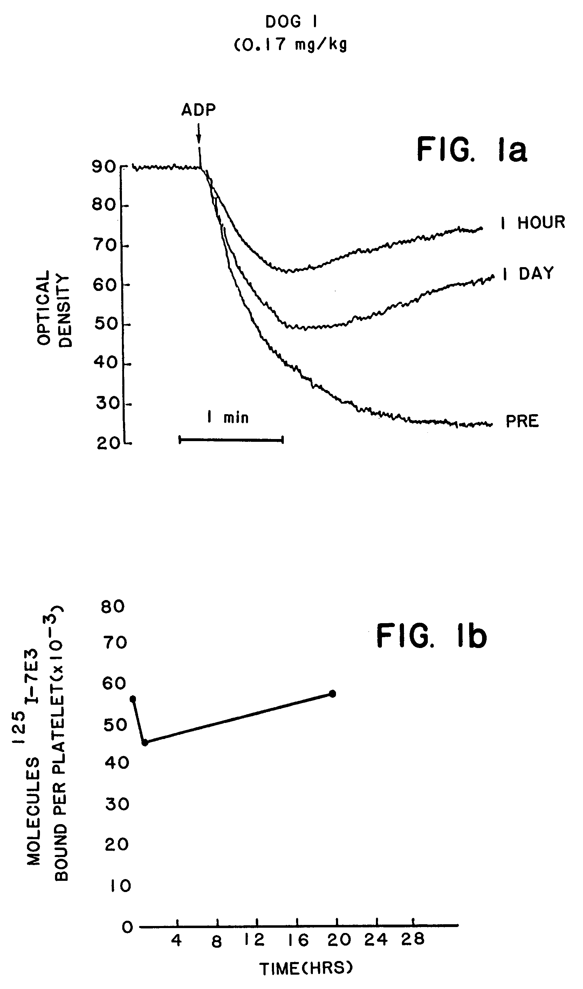

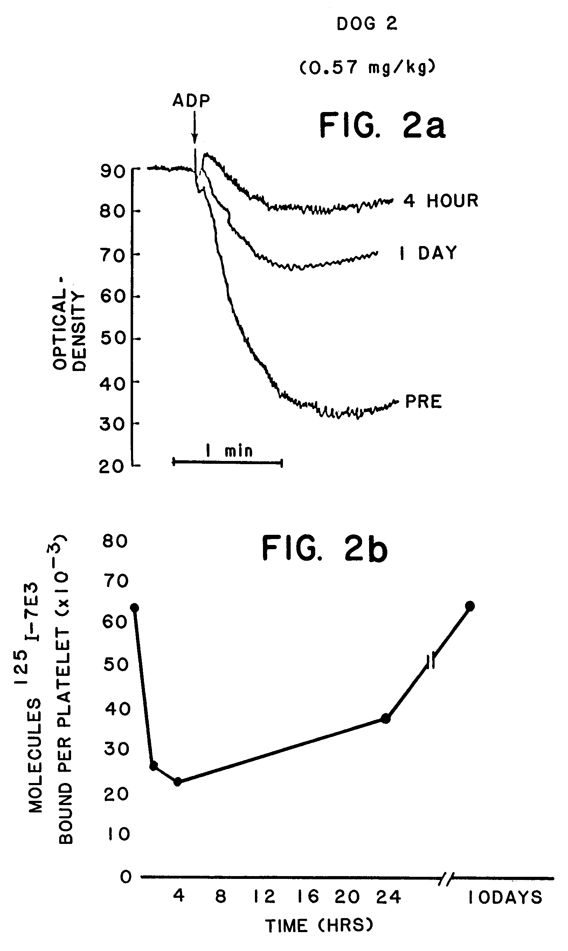

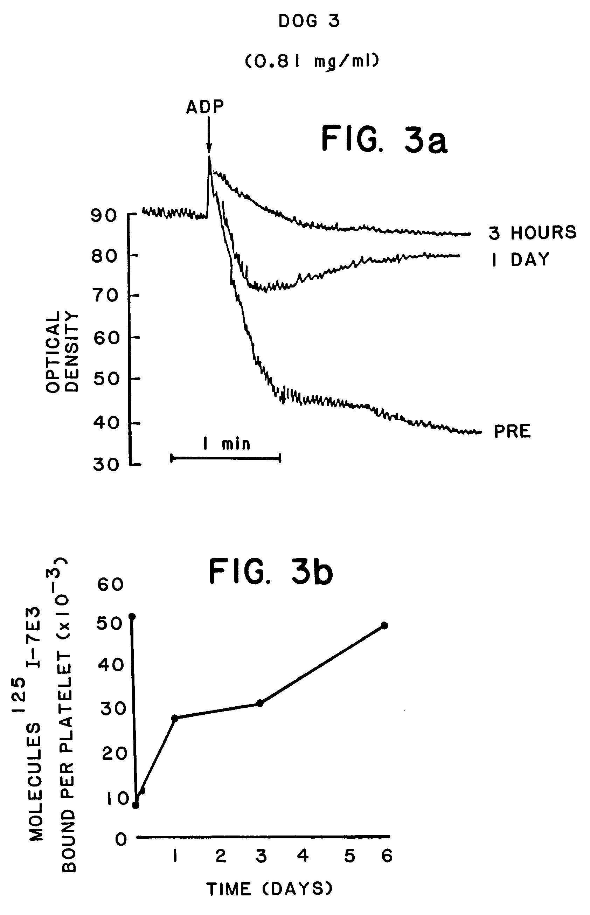

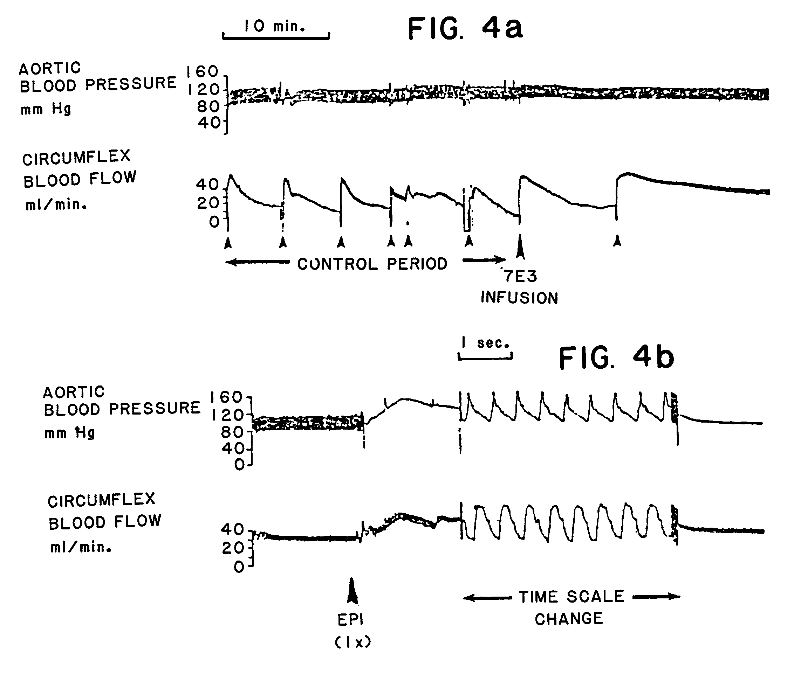

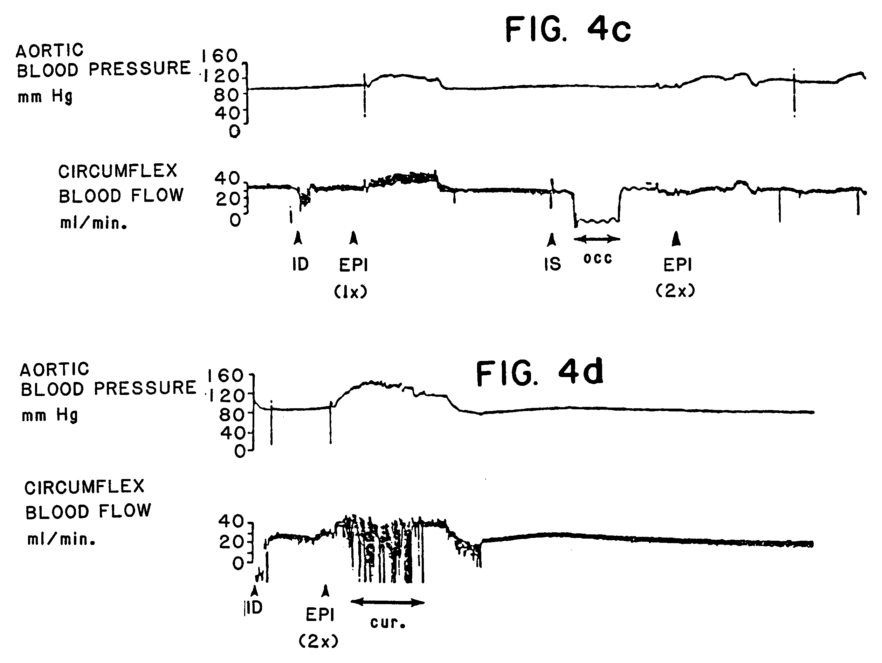

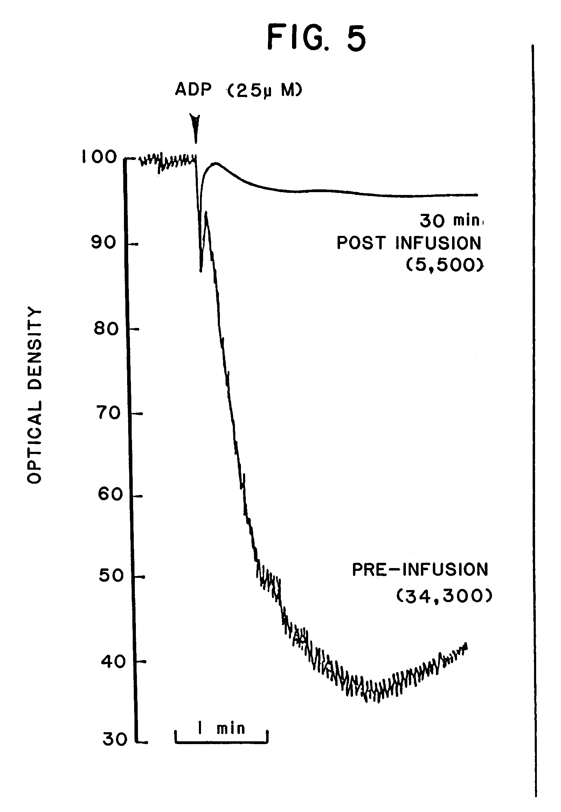

## Claims (Claims for the following Contracting State(s): BE, CH, DE, FR, GB, IT, LI, LU, NL, SE)

1. A monoclonal antibody fragment which has an antigen binding site and is derivable from a monoclonal antibody of the class IgG1 obtainable from a hybridoma of cells from a mouse myeloma line and spleen cells from a mouse previously immunized with human blood platelets, which antibody fragment
(a) inhibits platelet function;
(b) inhibits thrombosis;
(c) reacts with normal human platelets and with dog blood platelets;
(d) fails to react with thrombasthenic human platelets or human platelets whose GPIIb/IIIa complex is disassociated with EDTA;
(e) reacts slowly with unactivated platelets and more rapidly with ADP activated platelets, and
(f) blocks the interaction of fibrinogen with platelets induced by ADP.

2. A fragment according to claim 1 wherein the hybridoma is obtainable by fusion of X63-Ag 8.653 Balb/c murine myeloma cells and spleen cells from a Balb/c mouse.

3. A fragment according to claim 1 or claim 2 which is an F(ab')₂ fragment or an Fab fragment.

4. A fragment according to claim 3 which is an F(ab')₂ fragment.

5. A fragment according to any one of the preceding claims wherein the hybridoma is designated 7E3 having ATCC deposit number HB8832.

6. A pharmaceutical composition which comprises a therapeutically effective amount of a fragment according to any one of the preceding claims.

7. A method which comprises the use of a fragment according to any one of claims 1 to 5 to prepare a medicament for the treatment or prevention of platelet aggregation.

8. A method for producing a fragment according to any one of claims 1 to 5 which comprises the sequential steps of
i) immunizing mice with human blood platelets,
ii) removing the spleens from said mice and making a suspension of the said spleen cells,
iii) fusing said spleen cells with mouse myeloma cells in the presence of a fusion promoter,
iv) diluting and culturing the fused cells in separate wells in a medium which will not support the unfused myeloma cells,
v) evaluating the supernatant in each well containing a hybridoma for the presence of antibody to the fibrinogen receptor,
vi) selecting and cloning a hybridoma producing antibody that:
(a) inhibits platelet function;
(b) inhibits thrombosis;
(c) reacts with normal human platelets and with dog blood platelets;
(d) fails to react with thrombasthenic human platelets or human platelets whose GPIIb/IIIa complex is disassociated with EDTA,
(e) reacts slowly with unactivated platelets and more rapidly with ADP activated platelets, and
(f) blocks the interaction of fibrinogen with platelets induced by ADP.
vii) recovering the antibody from the supernatant above said clones and/or transferring said clones intraperitoneally into mice and harvesting the malignant ascites or serum from said mice, which ascites or serum contains the desired antibody,
viii) digesting said antibody with a proteolytic agent selected from the group consisting of papain and pepsin,
ix) recovering said fragment from the digestion mixture.

## Claims (Claims for the following Contracting State(s): AT)

1. A method which comprises the preparation of a monoclonal antibody fragment which has an antigen binding site and is derivable from a monoclonal antibody of the class IgG1 obtainable from a hybridoma of cells from a mouse myeloma line and spleen cells from a mouse previously immunized with human blood platelets, which antibody fragment
(a) inhibits platelet function;
(b) inhibits thrombosis;
(c) reacts with normal human platelets and with dog blood platelets;
(d) fails to react with thrombasthenic human platelets or human platelets whose GPIIb/IIIa complex is disassociated with EDTA;
(e) reacts slowly with unactivated platelets and more rapidly with ADP activated platelets, and
(f) blocks the interaction of fibrinogen with platelets induced by ADP.

2. A method according to claim 1 wherein the hybridoma is obtainable by fusion of X63-Ag 8.653 Balb/c murine myeloma cells and spleen cells from a Balb/c mouse.

3. A method according to claim 1 or claim 2 wherein the fragment is an F(ab')₂ fragment or an Fab fragment.

4. A method according to claim 3 wherein the fragment is an F(ab')₂ fragment.

5. A method according to any one of the preceding claims wherein the hybridoma is designated 7E3 having ATCC deposit number HB8832.

6. A method which comprises the preparation of a pharmaceutical composition which comprises a therapeutically effective amount of a fragment obtainable according to any one of the preceding claims.

7. A method which comprises the use of a fragment obtainable according to any one of claims 1 to 5 to prepare a medicament for the treatment or prevention of platelet aggregation.

8. A method according to any one of claims 1 to 5 which comprises the sequential steps of
i) immunizing mice with human blood platelets,
ii) removing the spleens from said mice and making a suspension of the said spleen cells,
iii) fusing said spleen cells with mouse myeloma cells in the presence of a fusion promoter,
iv) diluting and culturing the fused cells in separate wells in a medium which will not support the unfused myeloma cells,
v) evaluating the supernatant in each well containing a hybridoma for the presence of antibody to the fibrinogen receptor,
vi) selecting and cloning a hybridoma producing antibody that:
(a) inhibits platelet function;
(b) inhibits thrombosis;
(c) reacts with normal human platelets and with dog blood platelets;
(d) fails to react with thrombasthenic human platelets or human platelets whose GPIIb/IIIa complex is disassociated with EDTA,
(e) reacts slowly with unactivated platelets and more rapidly with ADP activated platelets, and
(f) blocks the interaction of fibrinogen with platelets induced by ADP.
vii) recovering the antibody from the supernatant above said clones and/or transferring said clones intraperitoneally into mice and harvesting the malignant ascites or serum from said mice, which ascites or serum contains the desired antibody,
viii) digesting said antibody with a proteolytic agent selected from the group consisting of papain and pepsin,
ix) recovering said fragment from the digestion mixture.

## Patentansprüche (Patentansprüche für folgende(n) Vertragsstaat(en): BE, DE, FR, GB, CH, IT, LI, LU, NL, SE)

1. Monoklonal Antikörperfragment, das eine Antigenbindungsstelle aufweist und von einem monoklonalen Antikörper der Klasse IgG1 ableitbar ist, der von einem Hybridom aus Zellen von einer Maus-Myelomlinie und Milzzellen einer zuvor mit menschlichen Blutplättchen immunisierten Maus erhältlich ist, welches Antikörperfragment
(a) die Blutplättchenfunktion hemmt;
(b) Thrombose hemmt;
(c) mit normalen menschlichen Blutplättchen und mit Hunde-Blutplättchen reagiert;
(d) nicht mit thrombasthenischen menschlichen Blutplättchen oder menschlichen Blutplättchen reagiert, deren GPIIb/IIIa-Komplex mit EDTA disassoziiert ist;
(e) langsam mit nicht aktivierten Blutplättchen und rascher mit ADP-aktivierten Blutplättchen reagiert, und
(f) die durch ADP induzierte Wechselwirkung von fibrinogen mit Blutplättchen blockiert.

2. Fragment nach Anspruch 1, worin das Hybridom durch Fusion von X63-Ag 8.653 Balb/c-Mäuse-Myleomzellen und Milzzellen von einer Balb/c-Maus erhältlich ist.

3. Fragment nach Anspruch 1 oder 2, welche ein F(ab')₂-Fragment oder ein Fab-Fragment ist.

4. Fragment nach Anspruch 3, welche ein F(ab')₂-Fragment ist.

5. Fragment nach einem der vorhergehenden Ansprüche, worin das Hybridom als 7E3 mit der ATCC-Hinterlegungsnummer HB8832 bezeichnet wird.

6. Pharmazeutische Zusammensetzung, die eine therapeutisch wirksame Menge eines nach einem dervorhergehenden Ansprüche Fragments.

7. Verfahren, welches die Verwendung eines nach einem der Ansprüche 1 bis 5 Fragments zur Herstellung eines Medikaments zur Behandlung von oder Vorbeugung gegen Blutplättchenaggregation umfaßt.

8. Verfahren zur herstellen eines nach einem der Ansprüche 1 bis 5, Fragments das der Reihe nach folgende Schritte umfaßt:
i) das immunisieren von Mäusen mit menschlichen Blutplättchen,
ii) das Entfernen der Milz aus den genannten Mäusen und die Herstellung einer Suspension aus den genannten Milzzellen;
iii) das Verschmelzen der genannten Milzzellen mit Maus-Myelomzellen in Gegenwart eines Fusionspromotors,
iv) das Verdünnen und Kultivieren der verschmolzenen Zellen in getrennten Mäpfen in einem Medium, das die nichtverschmolzenen Myelomzellen nicht unterstützt,
v) das Bewerten des Oberstands in jedem ein Hybridom enthaltenden Napf hinsichtlich des Vorhandenseins von Antikörper gegen den Fibrinogenrezeptor,
vi) das Ausgewählen und Klonieren eines Hybridoms, das Antikörper erzeugt, der:
(a) die Blutplättchenfunktion hemmt;
(b) Thrombose hemmt;
(c) mit normalen menschlichen Blutplättchen und mit Hunde-Blutplättchen reagiert;
(d) nicht mit thrombasthenischen menschlichen Blutplättchen oder menschlichen Blutplättchen reagiert, deren GPIIb/IIIa-Komplex mit EDTA disassoziiert ist;
(e) langsam mit nicht aktivierten Blutplättchen und rascher mit ADP-aktivierten Blutplättchen reagiert, und
(f) die die durch ADP induzierte Wechselwirkung von Fibrinogen mit Blutplättchen blockiert;
vii) das Gewinnen des Antikörpers aus dem Oberstand über den genannten Klonen und/oder das Obertragen der genannten Klone intraperitoneal in Mäuse und das Ernten der malignen Aszites oder von Serum aus den genannte Mäusen, welche(s) Aszites oder Serum den gewünschten Antikörper enthält;
viii) das Digerieren des genannten Antikörpers mit einem proteolytischen Mittel, das aus der Gruppe ausgewählt ist, die aus Papain und Pepsin besteht,
ix) das Gewinnen des genannten Fragments aus der Digestionsmischung.

## Patentansprüche (Patentansprüche für folgende(n) Vertragsstaat(en): AT)

1. Verfahren, welches die Herstellung eines monoklonalen Antikörperfragments umfaßt, das eine Antigenbindungsstelle aufweist und von einem monoklonalen Antikörper der Klasse IgG1 ableitbar ist, der von einem Hybridom aus Zellen von einer Maus-Myelomlinie und Milzzellen einer zuvor mit menschlichen Blutplättchen immunisierten Maus erhältlich ist, welches Antikörperfragment
(a) die Blutplättchenfunktion hemmt;
(b) Thrombose hemmt;
(c) mit normalen menschlichen Blutplättchen und mit Hunde-Blutplättchen reagiert;
(d) nicht mit thrombasthenischen menschlichen Blutplättchen oder menschlichen Blutplättchen reagiert, deren GPIIb/IIIa-Komplex mit EDTA disassoziiert ist;
(e) langsam mit nicht aktivierten Blutplättchen und rascher mit ADP-aktivierten Blutplättchen reagiert, und
(f) die durch ADP induzierte Wechselwirkung von Fibrinogen mit Blutplättchen blockiert.

2. Verfahren nach Anspruch 1, worin das Hybridom durch Fusion von X63-Ag 8.653 Balb/c-Mäuse-Myleomzellen und Milzzellen von einer Balb/c-Maus erhältlich ist.

3. Verfahren nach Anspruch 1 oder 2, worin das Fragment ein F(ab')₂-Fragment oder ein Fab-Fragment ist.

4. Verfahren nach Anspruch 3, worin das Fragment ein F(ab')₂-Fragment ist.

5. Verfahren nach einem der vorhergehenden Ansprüche, worin das Hybridom als 7E3 mit der ATCC-Hinterlegungsnummer HB8832 bezeichnet wird.

6. Verfahren, welches die Herstellung einer pharmazeutischen Zusammensetzung umfaßt, die eine therapeutisch wirksame Menge eines Fragments enthält, das nach einem dervorhergehenden Ansprüche erhältlich ist.

7. Verfahren, welches die Verwendung eines nach einem der Ansprüche 1 bis 5 erhältlichen Fragments zur Herstellung eines Medikaments zur Behandlung von oder Vorbeugung gegen Blutplättchenaggregation umfaßt.

8. Verfahren nach einem der Ansprüche 1 bis 5, das der Reihe nach folgende Schritte umfaßt:
i) das Immunisieren von Mäusen mit menschlichen Blutplättchen,
ii) das Entfernen der Milz aus den genannten Mäusen und die Herstellung einer Suspension aus den genannten Milzzellen;
iii) das Verschmelzen der genannten Milzzellen mit Maus-Myelomzellen in Gegenwart eines Fusionspromotors,
iv) das Verdünnen und Kultivieren der verschmolzenen Zellen in getrennten Näpfen in einem Medium, das die nichtverschmolzenen Myelomzellen nicht unterstützt,
v) das Bewerten des Überstands in jedem ein Hybridom enthaltenden Napf hinsichtlich des Vorhandenseins von Antikörper gegen den Fibrinogenrezeptor,
vi) das Ausgewählen und Klonieren eines Hybridoms, das Antikörper erzeugt, der:
(a) die Blutplättchenfunktion hemmt;
(b) Thrombose hemmt;
(c) mit normalen menschlichen Blutplättchen und mit Hunde-Blutplättchen reagiert;
(d) nicht mit thrombasthenischen menschlichen Blutplättchen oder menschlichen Blutplättchen reagiert, deren GPIIb/IIIa-Komplex mit EDTA disassoziiert ist;
(e) langsam mit nicht aktivierten Blutplättchen und rascher mit ADP-aktivierten Blutplättchen reagiert, und
(f) die die durch ADP induzierte Wechselwirkung von Fibrinogen mit Blutplättchen blockiert;
vii) das Gewinnen des Antikörpers aus dem Überstand über den genannten Klonen und/oder das Übertragen der genannten Klone intraperitoneal in Mäuse und das Ernten der malignen Aszites oder von Serum aus den genannte Mäusen, welche(s) Aszites oder Serum den gewünschten Antikörper enthält;
viii) das Digerieren des genannten Antikörpers mit einem proteolytischen Mittel, das aus der Gruppe ausgewählt ist, die aus Papain und Pepsin besteht,
ix) das Gewinnen des genannten Fragments aus der Digestionsmischung.

## Revendications (Revendications pour l'(les) Etat(s) contractant(s) suivant(s): BE, DE, FR, GB, CH, IT, LI, LU, NL, SE)

1. Fragment d'anticorps monoclonal qui a un site de liaison d'antigène et est dérivable d'un anticorps monoclonal de la classe IgG1 qui peut être obtenu à partir d'un hybrodome de cellules provenant d'une ligne de myélomes de souris et de cellules de rate provenant d'une souris antérieurement immuniséé avec des plaquettes de sang humain, lequel fragment d'anticorps
(a) inhibe la fonction des plaquettes ;
(b) inhibe la thrombose ;
(c) réagit avec les plaquettes humaines normales et avec les plaquettes du sang de chien ;
(d) échoue à réagir avec les plaquettes humaines thrombasthéniques ou les plaquettes humaines dont le complexe GPIIb/IIIa est dissoccié avec l'EDTA ;
(e) réagit lentement avec les plaquettes inactivées et plus rapidement avec les plaquettes activées par ADP, et
(f) bloque l'intéraction des fibrinogènes avec les plaquettes induites par l'ADP.

2. Fragment selon la revendication 1 dans laquelle l'hybridome peut être obtenu par fusion de cellules de myélome murine X63-Ag 8./653 Balb/c et de cellules de rate provenant d'une souris Balb/c.

3. Fragment selon la revendication 1 ou 2 qui est un fragment F(ab')₂ ou un fragment Fab.

4. Fragment selon la revendication 3 qui est un fragment F(ab')₂.

5. Fragment selon l'une quelconque des revendications précédentes dans laquelle l'hybridome est désigné 7E3 ayant un numéro de dépôt ATCC HB8832.

6. Composition pharmaceutique qui comprend une quantité thérapeutiquement efficace d'un fragment selon l'une quelconque des revendications précédentes.

7. Méthode qui comprend l'utilisation d'un fragment selon l'une quelconque des revendications 1 à 5 pour préparer un médicament pour la traitement ou la prévention de l'aggrégation des plaquettes.

8. Méthode de préparation d'un fragment selon l'une quelconque des revendications 1 à 5 qui comprend les étapes séquentielles de :
i) immunisation de souris avec des plaquettes de sang humain,
ii) élimination des rates desdites souris et fabrication d'une suspension desdites cellules de rate,
iii) fusion desdites cellules de rate avec les cellules de myélome de souris en présence d'un promoteur de fusion,
iv) dilution et culture des cellules fusionnées dans des puits séparés dans un milieu qui ne supportera pas les cellules de myélome non fusionnées,
v) évaluation du surnageant dans chaque puits contenant un hybridome quant à la présence d'un anticorps au récepteur fibrinogène,
vi) sélection et clonage d'un hybridome produisant l'anticorps qui :
(a) inhibe la fonction des plaquettes ;
(b) inhibe le thrombose ;
(c) réagit avec les plaquettes de sang normal et avec les plaquettes de sang de chien ;
(d) ne réagissent pas avec les plaquettes humaines thrombasthéniques ou les plaquettes humaines dont le complexe GPIIb/IIIa est dissocié avec l'EDTA,
(e) réagit lentement avec les plaquettes non activées et plus rapidement avec les plaquettes activées par l'ADP, et
(f) bloque l'intéraction de fibrinogène avec les plaquettes induites par l'ADP.
vii) récupération de l'anticorps surnageant et/ou transfert desdits clones de façon intrapéritonéalle dans des souris et récolte des ascites malins ou du sérum provenant desdites souris, lesquels ascites ou sérum contiennent les anticorps désirés,
viii) digestion dudit anticorps avec un agent protéolytique choisi parmi le groupe consistant de la papaïne et de la pepsine,
ix) récupération dudit fragment du mélange de digestion.

## Revendications (Revendications pour l'(les) Etat(s) contractant(s) suivant(s): AT)

1. Méthode qui comprend la préparation d'un fragment d'anticorps monoclonal qui a un site de liaison d'antigène et est dérivable d'un anticorps monoclonal de la classe IgG1 qui peut être obtenu à partir d'un hybrodome de cellules provenant d'une ligne de myélomes de souris et de cellules de rate provenant d'une souris antérieurement immunisée avec des plaquettes de sang humain, lequel fragment d'anticorps
(a) inhibe la fonction des plaquettes ;
(b) inhibe la thrombose ;
(c) réagit avec les plaquettes humaines normales et avec les plaquettes du sang de chien ;
(d) échoue à réagir avec les plaquettes humaines thrombasthéniques ou les plaquettes humaines dont le complexe GPIIb/IIIa est disoccié avec l'EDTA ;
(e) réagit lentement avec les plaquettes inactivées et plus rapidement avec les plaquettes activées par ADP, et
(f) bloque l'intéraction des fibrinogènes avec les plaquettes induites par l'ADP.

2. Méthode selon la revendication 1 dans laquelle l'hybridome peut être obtenu par fusion de cellules de myélome murine X63-Ag 8./653 Balb/c et de cellules de rate provenant d'une souris Balb/c.

3. Méthode selon la revendication 1 ou 2 dans laquelle le fragment est un fragment F(ab')₂ ou un fragment Fab.

4. Méthode selon la revendication 3 dans laquelle le fragment est un fragment F(ab')₂.

5. Méthode selon l'une quelconque des revendications précédentes dans laquelle l'hybridome est désigné 7E3 ayant un numéro de dépôt ATCC HB8832.

6. Méthode qui comprend la préparation d'une composition pharmaceutique qui comprend une quantité thérapeutiquement efficace d'un fragment qui peut être obtenu selon l'une quelconque des revendications précédentes.

7. Méthode qui comprend l'utilisation d'un fragment qui peut être obtenu selon l'une quelconque des revendications 1 à 5 pour préparer un médicament pour le traitement ou la prévention de l'aggrégation des plaquettes.

8. Méthode selon l'une quelconque des revendications 1 à 5 qui comprend les étapes séquentielles de :
i) immunisation de souris avec des plaquettes de sang humain,
ii) élimination des rates desdites souris et fabrication d'une suspension desdites cellules de rate,
iii) fusion desdites cellules de rate avec les cellules de myélome de souris en présence d'un promoteur de fusion,
iv) dilution et culture des cellules fusionnées dans des puits séparés dans un milieu qui ne supportera pas les cellules de myélome non fusionnées,
v) évaluation du surnageant dans chaque puits contenant un hybridome quant à la présence d'un anticorps au récepteur fibrinogène,
vi) sélection et clonage d'un hybridome produisant l'anticorps qui :
(a) inhibe la fonction des plaquettes ;
(b) inhibe la thrombose ;
(c) réagit avec les plaquettes de sang normal et avec les plaquettes de sang de chien ;
(d) ne réagissent pas avec les plaquettes humaines thrombasthéniques ou les plaquettes humaines dont le complexe GPIIb/IIIa est dissocié avec l'EDTA,
(e) réagit lentement avec les plaquettes non activées et plus rapidement avec les plaquettes activées par l'ADP, et
(f) bloque l'intéraction de fibrinogène avec les plaquettes induites par l'ADP.
vii) récupération de l'anticorps surnageant et/ou transfert desdits clones de façon intrapéritonéalle dans des souris et récolte des ascites malins ou du sérum provenant desdites souris, lesquels ascites ou sérum contiennent les anticorps désirés,
viii) digestion dudit anticorps avec un agent protéolytique choisi parmi le groupe consistant de la papaïne et de la pepsine,
ix) récupération dudit fragment du mélange de digestion.
